# EUROPEAN PATENT APPLICATION

(11) **EP 3 626 219 A1**
(43) Date of publication of application: **25.03.2020**
(21) Application number: 18196386.9
(22) Date of filing: 24.09.2018
(51) Int. Cl.: A61F 13/53, A41D 31/00, A61F 13/84, D06C 7/00, A41D 13/002, A61F 13/15, F28D 20/02

(54) **SUPERABSORBENT EVAPORATIVE COOLING FABRIC WITH IMPROVED STRUCTURAL INTEGRITY**

(71) Applicant: TechNiche International, Vista, CA 92081 (US)
(72) Inventor: FROST, Douglas, Vista, CA California 92081 (US); ANASTASAKIS, Ioannis, Vista, CA California 92081 (US)
(74) Representative: HGF Limited

(57) **Abstract**

An evaporative cooling fabric that can be fashioned into garments and accessories. The cooling fabric has a multi-layer design, which includes superabsorbent material configured to increase the water absorbing capacity of the fabric. The superabsorbent material includes a highly tangled, fibrous matrix including both hydrophilic and hydrophobic fibers. The evaporative cooling fabric provides structural integrity, which increases its useful life, improves temperature regulating performance, and makes it durable enough to withstand multiple machine washes.

## Description

### Field of the Invention

The present invention relates to evaporative cooling fabrics that can be fashioned into garments and accessories to be used for body cooling in humans and other mammals. The evaporative cooling fabric incorporates a multi-layer design including a superabsorbent material to increase the water absorbing capacity of the fabric. The superabsorbent material includes a highly tangled, fibrous matrix including both hydrophilic and hydrophobic fibers. The evaporative cooling fabric has improved structural integrity, which increases the useful life of the fabric, improves the temperature regulating performance and makes it durable enough to be machine washable.

### Background of the Invention

Evaporative cooling fabrics and garments made of such fabrics are used for thermal management of people or animals living or working in a natural environment or a work environment subject to elevated temperatures. Evaporative cooling fabrics and garments typically include an absorbent material that absorbs water and then allows the absorbed water to evaporate over an extended period of time to dissipate body heat and environmental heat through the latent heat of evaporation. Evaporative cooling fabrics and garments can improve the performance, safety and comfort of individuals operating in elevated temperature conditions. Heat tolerance can be extended and work productivity can be improved, while negative health consequences, such as heat stress and dehydration, can be reduced and minimized. Evaporative cooling fabrics can also be used for thermal management of heat-sensitive mechanical, electrical or electronic components, devices or assemblies.

Superabsorbent materials can be used to greatly increase the water absorbing capacity of an evaporative cooling fabric. A superabsorbent material can typically absorb 50-500 times its own weight in water. The superabsorbent material does not readily release liquid water that is absorbed into it, but it releases the water gradually as water vapor. Prior evaporative cooling fabrics that incorporate superabsorbent materials suffered from a limited useful life span. Particles or fibers of the superabsorbent material gradually migrate out of the fabric as it is subjected to repeated wet and dry cycles. The deterioration of an evaporative cooling fabric is greatly accelerated by machine washing because the fabric is also subjected to detergents, agitation and high centrifugal forces during the washing cycle.

The present invention overcomes these limitations of the prior art and provides a superabsorbent evaporative cooling fabric with improved structural integrity, which increases the useful life of the fabric, enhances its performance and makes it durable enough to be machine washable.

### Summary of the Invention

The evaporative cooling fabric of the present invention is a multilayer, multicomponent material. The evaporative cooling fabric has a nonwoven absorbent core layer that incorporates a multiplicity of carrier fibers, thermally bondable bicomponent fibers and superabsorbent fibers that are randomly laid or spun and subsequently intertangled and thermally bonded to create a cohesive nonwoven felt layer. The various fibers are multi-length which helps with structural integrity during the entanglement process. In addition to the absorbent core layer, the evaporative cooling fabric has a first nonabsorbent encasement layer that is bonded to a first surface of the core layer and a second nonabsorbent encasement layer that is bonded to a second surface of the core layer. The first and second encasement layers are carded and thermobonded webs, with a polyethylene coating on one side. They are bonded to the absorbent core layer using lamination, which imparts heat and pressure to melt the polyethylene coating of the carded webs and fuse to the absorbent core layer. Alternatively, the first and second encasement layers can be laid or spun directly onto the nonwoven absorbent core layer. The core layer is then sandwiched between inner and outer fabrics such as nylon, polyester, cotton or other woven or nonwoven fabrics.

The fibers of the absorbent core layer and the first and second encasement layers are intertangled using carding, needle punching, air entanglement, or other known felt making techniques to increase the structural integrity of the fabric. To further increase the structural integrity of the fabric, the three-layer structure is then subjected to heat, and optionally to pressure, to fuse the bicomponent fibers in the nonwoven absorbent core layer and in the first and second nonabsorbent layers, thus bonding the fibers of all three layers into a cohesive, interconnected fabric. The superabsorbent fibers are effectively encased or encapsulated within a strong, but flexible matrix formed by the carrier fibers and the bicomponent fibers, as well as the first and second encasement layers. The multilayer fabric formed by this multistep process has sufficient structural integrity to endure many wet and dry cycles, as well as to stand up to repeated machine washing.

Optionally, the outer fabric layers may be sewn or quilted to the three-layer structure of the absorbent core layer and the first and second encasement layers. The first and second outer fabric layers may be a woven or nonwoven material and may have other properties to enhance the evaporative cooling fabric. When the evaporative cooling fabric is fashioned into a garment, the edges of the evaporative cooling fabric may be bound with binding tape to create a finished edge and to seal in the superabsorbent fibers at the cut edges of the fabric.

### Brief Description of the Drawings

The above and related objects, features and advantages of the present invention will be more fully understood by reference to the following, detailed description of the preferred, albeit illustrative, embodiment of the present invention when taken in conjunction with the accompanying figures, wherein:
FIG. 1 shows an outer surface of a first example of an evaporative cooling fabric according to the present invention. The evaporative cooling fabric is cut away to show the internal structure of the fabric.
FIG. 2 shows an inner surface of the evaporative cooling fabric of FIG. 1. The evaporative cooling fabric is cut away to show the internal structure of the fabric.
FIG. 3 shows a cross section of the evaporative cooling fabric of FIG. 1 in a dry state.
FIG. 4 shows a cross section of the evaporative cooling fabric of FIG. 1 in a wet state.
FIG. 5 is a micrograph of the cross section of the evaporative cooling fabric of FIG. 1 in a dry state.
FIG. 6 is a micrograph of the evaporative cooling fabric of FIG. 1 showing a surface of one of the nonabsorbent layers of multilayer the evaporative cooling fabric.
FIG. 7 shows an outer surface of a second example of an evaporative cooling fabric according to the present invention.
FIG. 8 shows an inner surface of the evaporative cooling fabric of FIG. 7.
FIG. 9 is a graph illustrating the absorbency of the evaporative cooling fabric of the present invention after multiple wet/dry cycles compared to the prior art evaporative cooling fabric.
FIG. 10 is a graph illustrating the absorbency of the evaporative cooling fabric of the present invention after multiple machine wash cycles compared to the prior art evaporative cooling fabric.

### Detailed Description of the Invention

The evaporative cooling fabric of the present invention is a multilayer, multicomponent material which includes multi-length fibers and a multi-process manufacturing process. The evaporative cooling fabric has a nonwoven absorbent core layer that incorporates a multiplicity of carrier fibers, thermally bondable bicomponent fibers and superabsorbent fibers that are randomly laid or spun and subsequently intertangled and thermally bonded to create a cohesive nonwoven felt layer. The nonwoven absorbent core layer can be dry laid or air laid from staple fibers (i.e. cut fibers) or it can be spun from continuous filaments. The ratio of carrier fibers, thermally bondable bicomponent fibers and superabsorbent fibers in the fabric can be varied to determine the absorbency, water vapor release rate, strength and durability of the evaporative cooling fabric. As a nonlimiting example, the ratios of fibers in the absorbent core layer maybe 20% superabsorbent fibers, 71% polyester carrier fibers and 9% bicomponent fibers.

Preferably, the fibers are randomly laid or spun onto a moving belt to form a batting or felt of the desired thickness. The optimal thickness is approximately 2 mm but can be made thinner or thicker depending on the desired fabric weight, absorbency needs and overall garment design details. The fabric can be delivered in normal fabric widths but can be made narrower or wider depending on the physical limitations of the manufacturing equipment. Alternatively, the fibers may be laid or spun in a predetermined pattern, such as transversely, longitudinally or in a zigzag pattern, to affect the properties, such as strength, elongation or expansion, of the absorbent felt core layer. The three different types of fibers may be evenly distributed throughout the core layer or, optionally, different regions may have varying ratios of the fibers for different material properties in those regions. Optionally, needle punching, mechanical agitation and air jets and/or water jets maybe applied at this step to further entangle the fibers and increase the functional strength of the nonwoven absorbent core layer.

In addition to the absorbent core layer, the evaporative cooling fabric has a first nonabsorbent encasement layer that is bonded to a first surface of the core layer and a second nonabsorbent encasement layer that is bonded to a second surface of the core layer. The first and second encasement layers have a low gel permeability to minimize loss or migration of superabsorbent material from the core layer when it is in the wet gel state. The first and second encasement layers can be derived from the full spectrum of low basis weight nonwoven fabric forming technologies (e.g. spun bonds, melt blown, spun melts, spun laid, spun laced, polyurethane, needlefelts and carded nonwovens. In a nonlimiting example, the first and second encasement layers can contain two types of polyester fibers with different melting points that are carded and thermally bonded to create a thin nonwoven web. This web is coated on one side with polyethylene to facilitate thermal bonding for lamination. Alternatively, the first and second encasement layers can be made from the same carrier fibers and bicomponent fibers that are used in the absorbent core layer. Other fibers such as viscose, cellulose, polyester, polypropylene, polyethylene and acrylic can be used to form the first and second encasement layers. The first and second encasement layers can be dry laid or wet laid from staple fibers or they can be spun from continuous filaments. The first and second encasement layers can be separately laid or spun to create a thin nonwoven web and then these layers can be subsequently laminated to the first and second surfaces of the core layer using heat and pressure. Preferably, the first and second encasement layers are approximately 0.10-0.15 mm thick, however the thickness can vary depending on the materials, process and machines used. Alternatively, the first and second encasement layers can be laid or spun directly onto the nonwoven absorbent core layer, for example using heat fusible fibers and/or particulates. In addition, the first and second encasement layers may receive hydrophobic treatment (e.g. fluorocarbon) to form a surface layer that has lower gel permeability to resist loss or migration of the superabsorbent material. The first and second encasement layers can have the same composition or each layer may have a unique composition to provide different properties to the two sides of the evaporative cooling fabric.

Thermally bondable bicomponent fibers, also known as bico fibers, are textile fibers that are made of a first material with a first melt temperature and a second material with a second melt temperature that is lower than the first melt temperature. The first material and the second material are typically coextruded together to form a single fiber. When the bicomponent fibers are heated to a temperature between the first and second melt temperatures, the second material melts, fusing the second material to adjacent fibers in the fiber matrix. Due to its higher melt temperature, the first material remains intact, maintaining the structure of the fiber matrix during the heating process. The arrangement of the first material and second material within the fiber can vary. Some of the geometries for the bicomponent fibers can include: concentric sheath/core, eccentric sheath/core, side-by-side, pie wedge and islands/sea arrangements.

In one exemplary embodiment of the evaporative cooling fabric, the bicomponent fibers have a concentric sheath/core geometry with an inner core of polypropylene coextruded with an outer sheath of polyethylene. The polypropylene inner core has a melt temperature of approximately 160-170 C, whereas the polyethylene outer sheath has a melt temperature of approximately 120-140 C. When the bicomponent fibers are heated above 140 C, the polyethylene outer sheath melts, fusing the bicomponent fibers with adjacent fibers in the fiber matrix, but the polypropylene inner core remains solid below 160 C. When the material cools, the polyethylene solidifies, binding the fibers into an interlinking three-dimensional fiber matrix.

The carrier fibers provide bulk and strength to the nonwoven absorbent core layer. The carrier fibers are preferably a long, nonabsorbent, hydrophobic, polymer fiber with a high tensile strength and a melt temperature above the second melt temperature of the bicomponent fibers. For example, a polyester fiber, such as polyethylene terephthalate or polybutylene terephthalate, can be used as the carrier fiber.

Various superabsorbent materials can be used in the superabsorbent fibers. In one exemplary embodiment of the evaporative cooling fabric, long superabsorbent Polyacrylate fibers are used. Fiber length can range from 20 mm to 80 mm. In a further example, the superabsorbent Polyacrylate fiber is a cross-linked polymer consisting of three different monomers - acrylic acid, methylacrylate and a small quantity of special acrylate/methylacrylate monomer in which the acrylic acid is partially neutralized to the sodium salt of acrylic acid. The cross-links between polymer chains are formed as ester groups by reaction between the acid groups in acrylic acid and the special acrylate/methylacrylate monomer. This chemistry has been extensively tested to establish its "safe for use" status in application areas requiring low toxicity and/or regulatory approval. Studies include allergenicity, irritation, dermal, genetic, reproductive, and ingestion research.

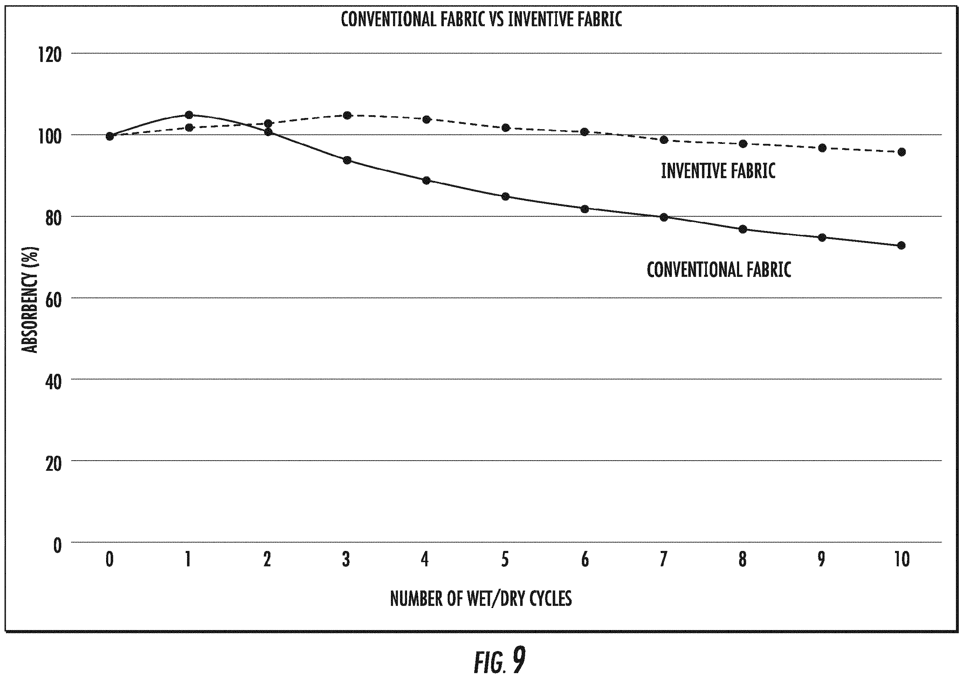

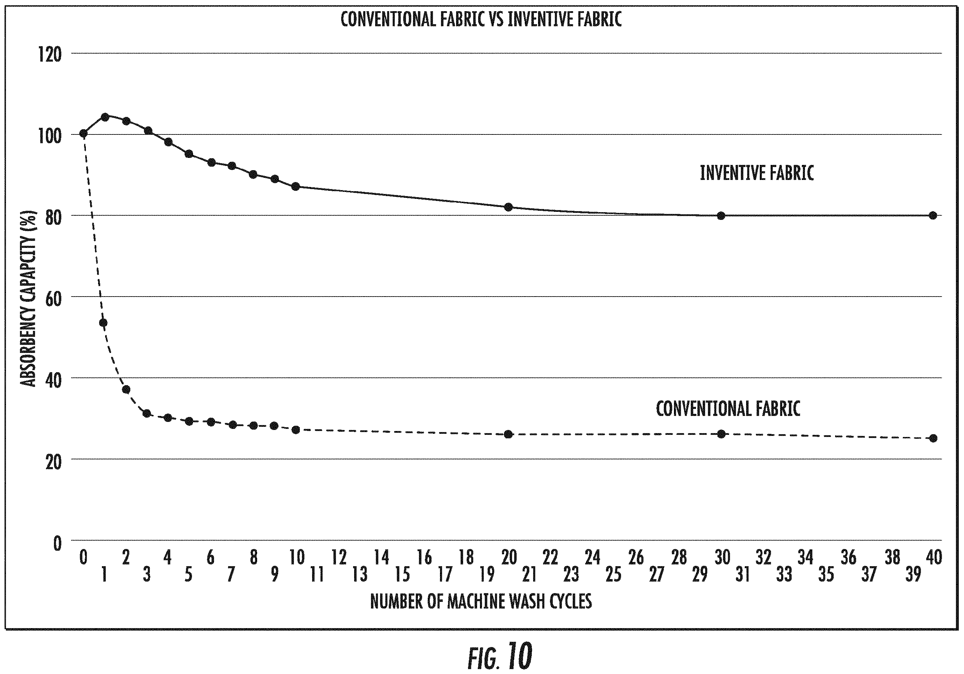

Various superabsorbent fibers have been used effectively in making the superabsorbent evaporative cooling fabric of the present invention. For example, the test data shown in FIGS. 9 and 10 below are from a superabsorbent evaporative cooling fabric made with superabsorbent fibers having a staple length of 50 mm and a decitex of 10 grams per 10,000 meters of fiber and having a moderate degree of cross linking characterized by a 15 minute free swell capacity of about 140 times its own weight of demineralized water. Additional examples of the superabsorbent evaporative cooling fabric have been made with superabsorbent fibers having a staple length of 50 mm and a decitex of 10 grams per 10,000 meters of fiber and having a higher degree of polymer cross linking characterized by a 15 minute free swell capacity of about 120 times its own weight of demineralized water. The degree of polymer cross linking can affect the performance of the superabsorbent fibers within the superabsorbent evaporative cooling fabric. The higher degree of polymer cross linking provides improved wet gel stability and integrity, thus reducing the propensity to gel shed during wet/dry and washing cycles and improving the durability of the superabsorbent evaporative cooling fabric.

The nonwoven absorbent felt core layer and the first and second encasement layers are assembled together as described above, then this three-layer structure goes through additional process steps to increase the structural integrity of the fabric. Needle punching, also known as needle felting, is performed with a machine that has rows of barbed or notched needles that pierce through the fabric multiple times. The barbs or notches catch some of the fibers and pull on them so that they become entwined or entangled with the surrounding fibers. The entanglement of the fibers within and between the layers increases the structural integrity of the each layer and of the three-layered fabric as a whole. The entanglement process can be accomplished in a variety of ways but must not lead to an over-compressed fabric as this will reduce the absorbency capabilities. Alternatively or in addition, mechanical agitation and air jets and/or water jets (hydro entanglement) may be used to entangle the fibers within the evaporative cooling fabric.

After entanglement of the fibers by needle punching, the three-layer structure of the evaporative cooling fabric is then heated to a temperature above 140 C to melt the polyethylene outer sheath of the bicomponent fibers in the nonwoven absorbent felt core layer (and in the first and second encasement layers if used there as well). Upon cooling, the polyethylene outer sheath of the bicomponent fibers fuses to the adjacent fibers, creating a three-dimensionally interlinked fiber matrix. Pressure may be applied to the three-layer structure during heating. Applied pressure increases the degree of interlinking between the fibers, which increases the strength and structural integrity of the fabric, and may also limit the expansion of the fabric when absorbing water. Pressure and heat may be applied with heated platens or rollers. Textured platens or rollers may be used to apply pressure and heat in a desired pattern.

Optionally, the evaporative cooling fabric may also include an outer fabric layer and an inner fabric layer. The outer and inner fabric layers may be glued, welded, sewn or quilted to the three- layer structure of the absorbent core layer and the first and second nonabsorbent layers. The outer and inner fabric layers may be a woven or nonwoven material and may have other properties to enhance the evaporative cooling fabric. For example, various technical fabrics may be used to provide enhanced wicking properties, light and/or reflective colors, hydrophobic/hydrophilic treatments, durability enhancements, etc. When the evaporative cooling fabric is fashioned into a garment, the edges of the evaporative cooling fabric may be bound with binding tape to create a finished edge and to seal in the superabsorbent fibers at the cut edges of the fabric.

The following numbered statements 1-28 are not claims, but instead serve to define particular aspects and embodiments of the claimed invention:
1. A superabsorbent evaporative cooling fabric with improved structural integrity, comprising:
   an absorbent core layer comprising a nonwoven matrix of:
      carrier fibers;
      bicomponent fibers made of a first material with a first melt temperature and a second material with a second melt temperature that is lower than the first melt temperature; and
      superabsorbent fibers;
   wherein the carrier fibers, bicomponent fibers and superabsorbent fibers are intertangled and bonded together to form a cohesive matrix;
   a first encasement layer bonded to a first surface of the absorbent core layer comprising a nonwoven web of nonabsorbent fibers; and
   a second encasement layer bonded to a second surface of the absorbent core layer comprising a nonwoven web of nonabsorbent fibers.
2. The cooling fabric of statement 1, where the carrier fibers are polyester fibers.
3. The cooling fabric of statement 1 or 2, where the bicomponent fibers have an inner core of polypropylene surrounded by a sheath of polyethylene.
4. The cooling fabric of statement 3 where the polypropylene core has a melt temperature of approximately 160-170 C and the polyethylene sheath has a melt temperature of approximately 120-140 C.
5. The cooling fabric of any one of statements 1-4, wherein the superabsorbent fibers are cross-linked polyacrylate fibers.
6. The cooling fabric of any one of statements 1-5, where ratios of fibers in the absorbent core layer is approximately 20% superabsorbent fibers, 71% polyester carrier fibers and 9% bicomponent fibers.
7. The cooling fabric of any one of statements 1-6 where the bicomponent fibers are thermally bondable.
8. The cooling fabric of statement 7 where the carrier fibers, thermally bondable bicomponent fibers, and superabsorbent fibers are thermally bonded to form the absorbent core layer.
9. The cooling fabric of any one of statements 1-8, where the absorbent core layer and the first and second encasement layers are fused such that absorbent core layer and the first and second encasement layers form a cohesive, interconnected fabric.
10. The cooling fabric of any one of statements 1-9 where the first and second encasement layers are intertangled with the absorbent core layer.
11. The cooling fabric of any one of statements 1-10, having a first fabric layer sewn to the first and encasement layer and a second fabric layer sewn to the second encasement layer.
12. The cooling fabric of any one of statements 1-11 where the first encasement layer is laid or spun directly onto a first surface of the cohesive non woven core layer and the second encasement layer is laid or spun directly onto a second surface of the cohesive non woven core layer.
13. The cooling fabric of statement 1 where each of the first and second encasement layers is a carded thermobonded web having a polyethylene coating on one side.
14. The cooling fabric of any one of statements 1-13 where the first and second encasement layers are each formed of two types of polyester fibers with different melting points that are carded and thermally bonded to create a thin nonwoven web.
15. The cooling fabric of any one of statements 1-14 where the first and second encasement layers are formed of the same carrier fibers and bicomponent fibers that are used in the absorbent core layer.
16. The cooling fabric of any one of statements 1-15 where the first and second encasement layers are formed of fibers such as any of viscose, cellulose, polyester, polypropylene, polyethylene and acrylic.
17. The cooling fabric of any one of statements 1-16 where the first and second encasement layers are treated with hydrophobic material.
18. The cooling fabric of statement 11 where the first and second fabric layers are glued, welded, sewn or quilted to cohesive, interconnected fabric formed by the fused absorbent core layer and the first and second encasement layers.
19. The cooling fabric of statement 11 where the first and second fabric layers are formed of woven material.
20. The cooling fabric of statement 11 where the first and second fabric layers are formed of non-woven material.
21. A method of manufacturing the cooling fabric of any one of statements 1-20 comprising the steps of:
   providing a multiplicity of carrier fibers, thermally bondable bicomponent fibers, and superabsorbent fibers;
   randomly laying the carrier fibers, thermally bondable bicomponent fibers, and superabsorbent fibers onto a moving belt;
   entangling the carrier fibers, thermally bondable bicomponent fibers, and superabsorbent fibers;
   thermally bonding the carrier fibers, thermally bondable bicomponent fibers, and superabsorbent fibers to form a cohesive nonwoven absorbent core layer;
   providing a first encasement layer and a second encasement layer;
   coating a first surface of the first encasement layer and a first surface of the second encasement layer with polyethylene; and
   thermally bonding the first and second encasement layers to the first and second surfaces of the nonwoven absorbent core.
22. The method of statement 21 further comprising applying any one or more of needle punching, mechanical agitation and air jets and/or water jets to entangle the fibers and increase the functional strength of the nonwoven absorbent core layer.
23. The method of statement 21 or 22 further comprising the steps of deriving via a low basis weight nonwoven fabric forming technology (e.g. spun bonds, melt blown, spun melts, spun laid, spun laced) carded nonwovens forming a first encasement layer and a second encasement layer.
24. The method of any one of statements 21-23 further comprising the step of coating the first and second encasement layers with polyethylene to facilitate thermal bonding for lamination.
25. The method of any one of statements 21-24 further comprising the step of thermally bonding the first and second encasement layers to the first and second surfaces of the nonwoven absorbent core.
26. The method of any one of statements 21-25 further comprising the step of heating the absorbent core layer to a temperature above 140 C.
27. The method of statement 26 where the temperature is between 140 C and 160 C.
28. The method of statement 26 where the temperature is between 140 C and 170 C.

### Example 1

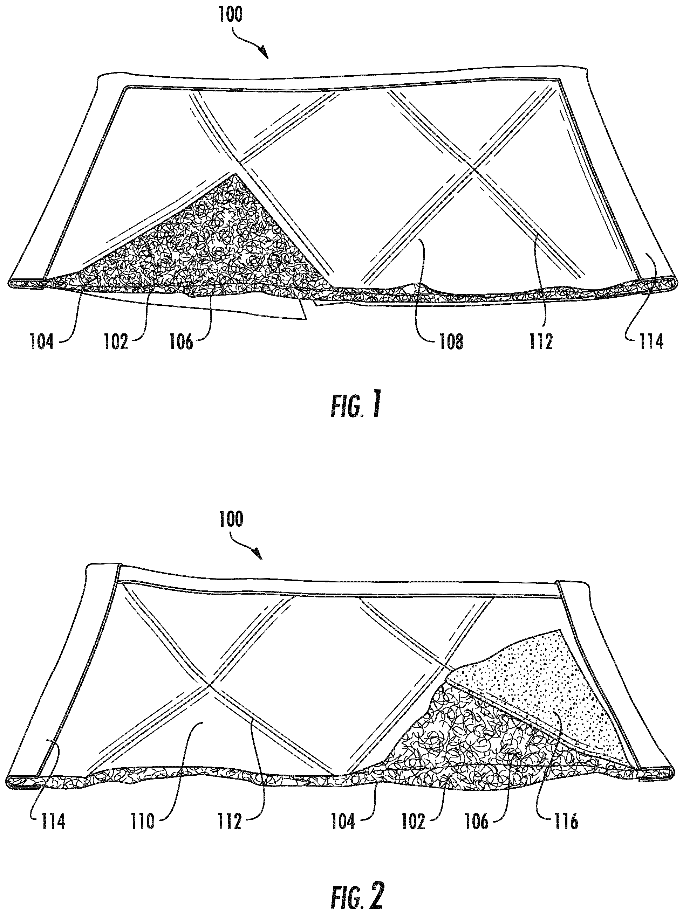

FIG. 1 shows an outer surface of a first example of an evaporative cooling fabric 100 according to the present invention. FIG. 2 shows an inner surface of the evaporative cooling fabric of FIG. 1. The outer surface of the evaporative cooling fabric is nominally the side that faces toward the environment and away from the skin of the wearer, and the inner surface of the evaporative cooling fabric is nominally the side that faces toward the skin of the wearer.

The evaporative cooling fabric 100 in FIGS. 1 and 2 is cut away to show the internal structure of the fabric. At the heart of the evaporative cooling fabric is a three-layered structure with a nonwoven absorbent felt core layer 102 and first 104 and second 106 nonabsorbent encasement layers bonded to the core layer 102. The outer surface of the evaporative cooling fabric is covered with an outer fabric layer 108 and the inner surface of the evaporative cooling fabric is covered with an inner fabric layer 110. The outer 108 and inner 110 fabric layers may be a woven or nonwoven material and may have other properties to enhance the evaporative cooling fabric. In the example shown, the outer fabric layer 108 is a coarse weave textile fabric that allows air, water and water vapor to flow to and from the absorbent core layer. Preferably, the outer fabric layer is a nonabsorbent material so that it provides a substantially dry outer surface to the evaporative cooling fabric, even when the absorbent core layer is saturated. Optionally, the outer fabric layer may include a hydrophobic treatment which enhances the rate of evaporative cooling. As a nonlimiting example, the outer fabric layer may be an oxford weave nylon material. The inner fabric layer may be a finer weave textile fabric that presents a smooth surface to the skin of the wearer. Preferably, the inner fabric layer is also a nonabsorbent material so that it provides a substantially dry inner surface to the skin of the wearer. Optionally, the inner fabric may be a water repellent or waterproof fabric designed to act as a water barrier between the hydrated fabric and the wearer. The inner fabric layer can be enhanced with a coating 116, for example PTFE (polytetrafluoroethylene) or polyurethane coating, which provide a breathable coating that acts as a barrier to liquid water, but allows water vapor to pass through. As a nonlimiting example, the inner fabric layer may be a fine ripstop weave nylon material with a PTFE coating. The outer and/or inner fabric layers may have other properties that contribute to the performance of the evaporative cooling fabric. For instance, in the example shown, the outer fabric layer 108 is a reflective silver color to reflect radiant heat from the surrounding environment, which contributes to thermal management.

As shown, the outer 108 and inner 110 fabric layers may be sewn or quilted 112 to the three- layer structure of the absorbent core layer 102 and the first 104 and second 106 nonabsorbent layers. When the evaporative cooling fabric 100 is fashioned into a garment or accessory, the edges of the evaporative cooling fabric may be bound with binding tape 114 to create a finished edge and to seal in the superabsorbent fibers at the cut edges of the fabric.

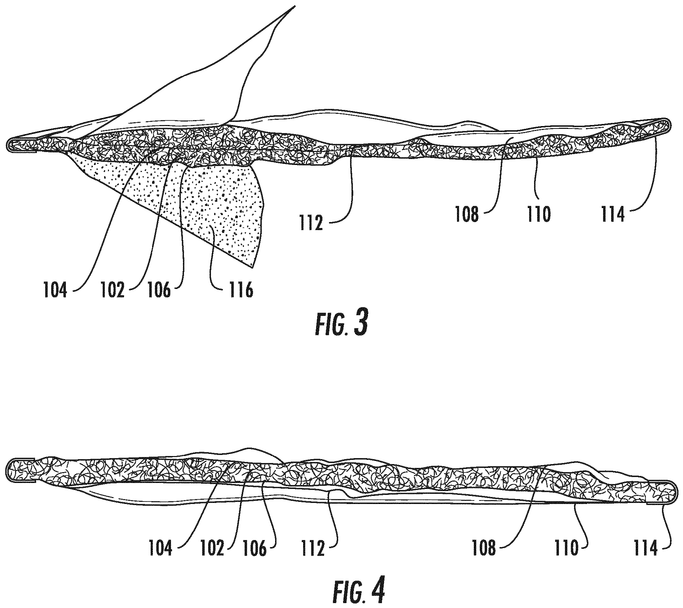

FIG. 3 shows a cross section of the evaporative cooling fabric 100 of FIG. 1 in a dry state, and FIG. 4 shows a cross section of the evaporative cooling fabric of FIG. 1 in a wet state. The superabsorbent fibers in the nonwoven absorbent felt core layer 102 swell as they absorb up to 50-500 times their own weight in water. The swelling of the nonwoven absorbent felt core layer 102 is somewhat limited by the structure of the evaporative cooling fabric 100 so that the garment does not become uncomfortable or ungainly due to excessive swelling.

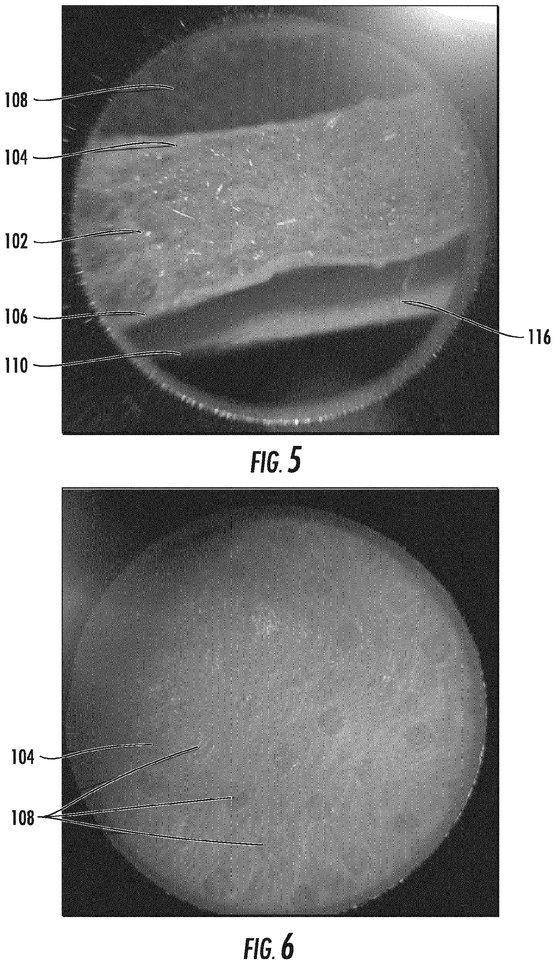

FIG. 5 is a micrograph of the cross section of the evaporative cooling fabric of FIG. 1 in a dry state. At the center of the evaporative cooling fabric is the nonwoven absorbent felt core layer, consisting of carrier fibers, thermally bondable bicomponent fibers and superabsorbent fibers. The first nonabsorbent encasement layer is shown on top of the core layer and the second nonabsorbent encasement layer is shown on the bottom of the core layer. The first and second nonabsorbent encasement layers consist of polyester fibers carded and thermally bonded, with a polyethylene coating on one side to facilitate lamination. The nonwoven absorbent felt core layer and the first and second nonabsorbent encasement layers are needle punched and then thermally bonded to improve the structural integrity.

FIG. 6 is a micrograph of the evaporative cooling fabric 100 of FIG. 1 showing a surface of one of the nonabsorbent encasement layers 104 of the multilayer evaporative cooling fabric. In the example shown, the nonwoven absorbent felt core layer and the first and second nonabsorbent encasement layers are thermally bonded using textured rollers. The thermal bonding pattern 118 from the textured rollers can be seen on the surface of the nonabsorbent felt layers 104 in FIG. 6.

### Example 2

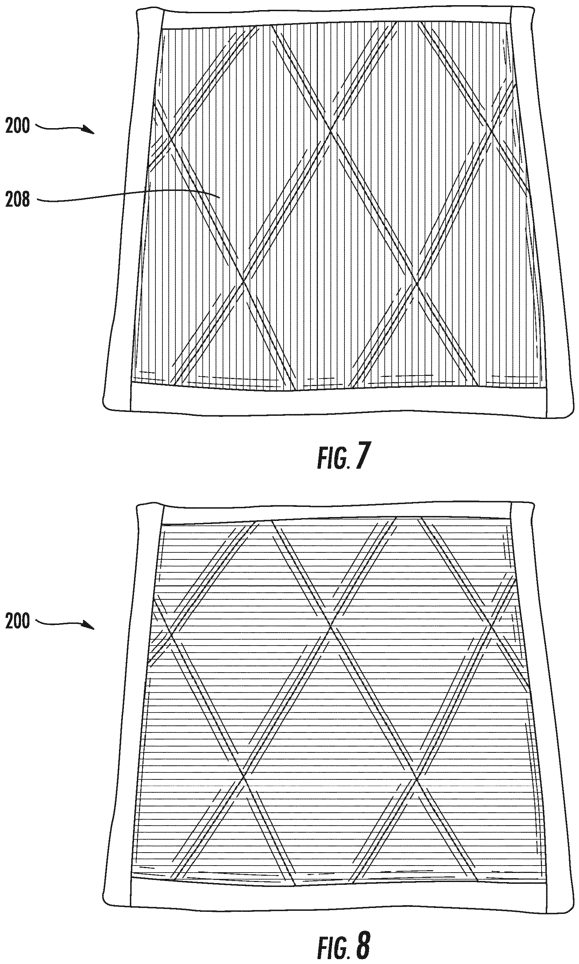

FIG. 7 shows an outer surface of a second example of an evaporative cooling fabric 200 according to the present invention. FIG. 8 shows an inner surface of the evaporative cooling fabric of FIG. 7. The internal structure of the evaporative cooling fabric 200 is the same as the first example described above, but the outer fabric layer 208 is selected for different properties.

In the example shown, the outer fabric layer 208 is a Taslan nylon for a softer hand and smoother texture, while still allowing airflow and passage of water vapor. The outer fabric layer is shown in "100% polyester safety orange" which provides high visibility for safety vests and similar garments.

### Method of Use

The evaporative cooling fabric of the present invention maybe fashioned into a wide variety of garments, including, but not limited to: vests, jackets, coats, shirts, trousers, shorts, overalls, coveralls, aprons, skirts, dresses, jumpers, gloves, mittens, hats, caps, hoods, scarves, face masks, socks, shoe covers, and animal vests or blankets. Alternatively, individual sheets or panels of evaporative cooling fabric can placed into a garment or otherwise fastened to the user.

Prior to use, the evaporative cooling fabric or garment is briefly soaked in water to saturate the nonwoven absorbent felt core layer. Loose drops of water can be shaken off or wiped from the outer surfaces of the garment before donning it. The water absorbed into the evaporative cooling fabric evaporates over an extended period of time. The period of effective cooling varies depending on the environmental conditions and the exertion of the user. A typically configured evaporative cooling fabric will last 4-8 hours (and oftentimes up to 12 hours) depending on the environmental conditions (e.g. humidity, ambient temp, air flow, etc).

FIGS. 9 and 10 are graphs illustrating the improved performance and durability of the evaporative cooling fabric according to the present invention. FIG. 9 is a graph illustrating the absorbency of the evaporative cooling fabric of the present invention after multiple wet/dry cycles compared to the conventional art evaporative cooling fabric. It can be seen from the graph that the absorption characteristics of the evaporative cooling fabric do not deteriorate significantly after repeated wet/dry cycles, as compared to the conventional art evaporative cooling fabric. The absorption characteristics correlate directly with the cooling effectiveness of the evaporative cooling fabric.

FIG. 10 is a graph illustrating the absorbency of the evaporative cooling fabric of the present invention after multiple machine wash cycles compared to the prior art evaporative cooling fabric. The testing was performed using normal machine wash cycles. The inventive evaporative cooling fabric has enough structural integrity to withstand repeated machine washing cycles without structural damage or deterioration of the absorption and therefore the cooling effectiveness. The superabsorbent material is prevented from migrating out of the evaporative cooling fabric during soaking or washing by the three-layered structure of the felt material that effectively encapsulates the superabsorbent material. The structural integrity of the inventive evaporative cooling fabric is further enhanced by the outer and inner fabric layers that are stitched or quilted onto the outside of the fabric and the edge binding.

While in the foregoing specification a detailed description of specific embodiments of the invention was set forth, it will be understood that many of the details herein given may be varied considerably by those skilled in the art without departing from the spirit and scope of the invention.

## Claims

1. A superabsorbent evaporative cooling fabric with improved structural integrity, comprising:
an absorbent core layer comprising a nonwoven matrix of:
carrier fibers;
bicomponent fibers made of a first material with a first melt temperature and a second material with a second melt temperature that is lower than the first melt temperature; and
superabsorbent fibers;
wherein the carrier fibers, bicomponent fibers and superabsorbent fibers are intertangled and bonded together to form a cohesive matrix;
a first encasement layer bonded to a first surface of the absorbent core layer comprising a nonwoven web of nonabsorbent fibers; and
a second encasement layer bonded to a second surface of the absorbent core layer comprising a nonwoven web of nonabsorbent fibers.

2. The superabsorbent evaporative cooling fabric of claim 1, wherein the carrier fibers comprise polyester fiber.

3. The superabsorbent evaporative cooling fabric of claim 1, wherein the bicomponent fibers comprise an inner core of polypropylene surrounded by a sheath of polyethylene.

4. The superabsorbent evaporative cooling fabric of claim 1, wherein the superabsorbent fibers comprise cross-linked polyacrylate fibers.

5. The superabsorbent evaporative cooling fabric of claim 1, wherein the absorbent core layer and the first and second encasement layers are fused by heat such that absorbent core layer and the first and second encasement layers form a cohesive, interconnected fabric.

6. The superabsorbent evaporative cooling fabric of claim 1, where ratios of fibers in the absorbent core layer is approximately 20% superabsorbent fibers, 71% polyester carrier fibers and 9% bicomponent fibers.

7. The superabsorbent evaporative cooling fabric of claim 1, further comprising an outer fabric layer and an inner fabric layer sewn to the first and second encasement layers.

8. A method of manufacturing a superabsorbent evaporative cooling fabric of any of Claims 1-7 comprising the steps of:
providing a multiplicity of carrier fibers, thermally bondable bicomponent fibers, and superabsorbent fibers;
randomly laying the carrier fibers, thermally bondable bicomponent fibers, and superabsorbent fibers onto a moving belt;
entangling the carrier fibers, thermally bondable bicomponent fibers, and superabsorbent fibers;
thermally bonding the carrier fibers, thermally bondable bicomponent fibers, and superabsorbent fibers to form a cohesive nonwoven absorbent core layer;
providing a first encasement layer and a second encasement layer;
coating a first surface of the first encasement layer and a first surface of the second encasement layer with polyethylene; and
thermally bonding the first and second encasement layers to the first and second surfaces of the nonwoven absorbent core.

9. The method of Claim 8 further comprising the step of applying any one or more of needle punching, mechanical agitation and air jets and/or water jets to entangle the fibers and increase the functional strength of the nonwoven absorbent core layer.

10. The method of Claim 9 further comprising the step of coating the first and second encasement layers with polyethylene to facilitate thermal bonding for lamination.

11. The method of Claim 10 further comprising the step of heating the absorbent core layer to a temperature between 140 C and 160 C.

12. The method of Claim 10 further comprising the step of heating the absorbent core layer to a temperature between 140 C and 170 C.
